# EUROPEAN PATENT APPLICATION

(11) **EP 4 640 179 A1**
(43) Date of publication of application: **29.10.2025**
(21) Application number: 24172855.9
(22) Date of filing: 26.04.2024
(51) Int. Cl.: A61C 13/00, G06F 30/10, G06T 17/00, G16H 30/40, G06T 19/20

(54) **COMPUTER-IMPLEMENTED METHODS, 3D CAD SYSTEM AND APPARATUSES FOR ESTIMATING ONE OR MORE SCALING FACTORS TO MAP BETWEEN A 2D DIGITAL IMAGE OF A DENTITION AND A 3D DIGITAL MODEL OF A DENTITION AND JAW**

(71) Applicant: Digicuto, 13008 Marseille (FR)
(72) Inventor: Koubi, Stefan, 13008 Marseille (FR); Gurel, Galip, 13008 Marseille (FR); Tourbah, Karim, 13008 Marseille (FR)
(74) Representative: A.P.I. Conseil

(57) **Abstract**

The subject application provides computer-implemented methods, a 3D CAD system and apparatuses for estimating one or more scaling factors to map between a 2D digital image of a dentition and a 3D digital model of a dentition and jaw.

The subject application provides a solution to the technical problem of accurately establishing a spatial and dimensional correlation between 2D and 3D dental imaging techniques, leading to improved patient outcomes and more efficient dental treatment planning.

It offers a more precise, efficient approach, thereby improving the process of preparing for a dental restoration.

## Description

### Technical Field

The subject application relates to computer-implemented methods, a 3D CAD system and apparatuses for estimating one or more scaling factors to map between a 2D digital image of a dentition and a 3D digital model of a dentition and jaw.

### Background Art

In the field of dental prosthetic design, the use of 2D and 3D dental imaging techniques offers significant advantages in terms of precision and detail.

However, accurately establishing a spatial and dimensional correlation between these imaging techniques presents a challenge, as the process often relies on manual mapping.

This manual method, due to its qualitative nature and dependence on human judgment, is susceptible to variability and errors.

These inconsistencies in correlating 2D and 3D dental images can lead to substantial challenges in applications such as prosthetic restoration design workflows.

A lack of precision in the mapping process can result in less accurate treatment plans, leading to less than optimal patient outcomes and potentially further complications.

In addition, these issues can increase the time and effort required for dental treatment planning, causing inconvenience for both patients and dental professionals.

These drawbacks highlight the need for a more accurate and efficient solution to the technical problem of mapping between 2D and 3D dental images.

### Summary of the subject application

As described in the accompanying claims, the subject application provides methods, systems and apparatuses for mapping between 2D and 3D dental images, correlating spatial and dimensional characteristics, and estimating scaling factors for enhanced dental treatment planning.

Dependent claims describe specific embodiments of the subject application.

These and other aspects of the subject application will be apparent from an elucidated based on the embodiments described hereinafter.

### Brief Description of Drawings

Further details, aspects and embodiments of the subject application will be described, by way of example only, with reference to the drawings. In the drawings, like reference numbers are used to identify like or functionally similar elements. Elements in the figures are illustrated for simplicity and clarity and have not necessarily been drawn to scale. Elements indicated by a solid line are mandatory, while elements indicated by a dotted line are optional.
Figure 1 shows a schematic flow diagram of a computer-implemented method according to the subject application.
Figure 2 shows a first view of the 3D digital model of the dentition and jaw of a patient.
Figure 3 shows a second view of the 3D digital model of the dentition and jaw of figure 4.
Figure 4 shows a virtual 2D digital image of the dentition of a patient, according to the subject application.
Figure 5 shows a synthetic 2D digital image of the dentition of a patient, according to the subject application.
Figure 6 shows the result of the positioning of a virtual 2D digital image of the dentition of a patient and 3D digital model of the dentition and jaw of a patient, according to the subject application.
Figure 7 shows several variations of the computer-implemented method of figure 1.
Figure 8 shows a block diagram of a system according to the subject application.
Figure 9 shows a block diagram of an apparatus according to the subject application.

### Description of Embodiments

Because the illustrated embodiments of the subject application may, for the most part, be composed of components known to the skilled person, details will not be explained in any greater extent than that considered necessary for the understanding and appreciation of the underlying concepts of the subject application, in order not to obfuscate or distract from the teachings of the subject application.

### ► Problem solved by the subject application

Inventors have found a way to address the issue of accurately establishing a spatial and dimensional correlation between 2D and 3D imaging techniques capturing the same anatomy.

The technical problem that the subject application seeks to solve is the challenge of manually mapping 2D dental images to corresponding 3D models, a process that has proven to be prone to variability and errors.

This lack of precision and standardization in correlating 2D and 3D digital dental representations reduces reliability and reproducibility in applications such as prosthetic restoration design workflows.

The solution to this technical problem would greatly enhance the analysis and integration of these complementary modalities, paving the way for a more robust and optimized dental treatment planning.

By improving the precision of 2D to 3D dental image registration, dental professionals could rely on a more standardized and quantitative mapping between 2D and 3D imaging data, leading to better patient outcomes.

### ►Solution of the subject application

The inventors have developed a computer-implemented method specifically intended for estimating one or more scaling factors to map between a 2D digital image of a dentition and a 3D digital model of a dentition and jaw.

This method represents a significant improvement in the process of dental treatment planning, offering a solution that is more accurate and less prone to errors compared to manual mapping.

Thus, the invention aims to greatly enhance the analysis and integration of 2D and 3D dental imaging modalities, leading to better patient outcomes and more efficient dental treatment planning.

In particular, the proposed method involves several steps.

Firstly, it acquires a 3D digital model of the patient's dentition and jaw which has been transformed to be centered, aligned with the X, Y, and Z axes of a CAD coordinate system.

Next, it identifies, using the 3D digital model, three or more first landmarks on the dentition and calculates first landmark-based geometric morphometrics.

Then, it acquires a virtual or synthetic 2D digital image of the patient's dentition and aligns the image with the X and Y axes of the CAD coordinate system.

It identifies, using the 2D digital image, three or more second landmarks on the dentition, which correspond to the same anatomical locations as the first landmarks determined in the 3D digital model, and calculates second landmark-based geometric morphometrics.

Finally, it estimates one or more scaling factors based on the comparison of the first and second landmark-based geometric morphometrics.

In conclusion, the proposed method provides a solution to the technical problem of accurately establishing a spatial and dimensional correlation between 2D and 3D dental imaging techniques, leading to improved patient outcomes and more efficient dental treatment planning.

It offers a more precise, efficient approach, thereby improving the process of preparing for a dental restoration.

### ►First aspect of the subject application: a first computer-implemented method

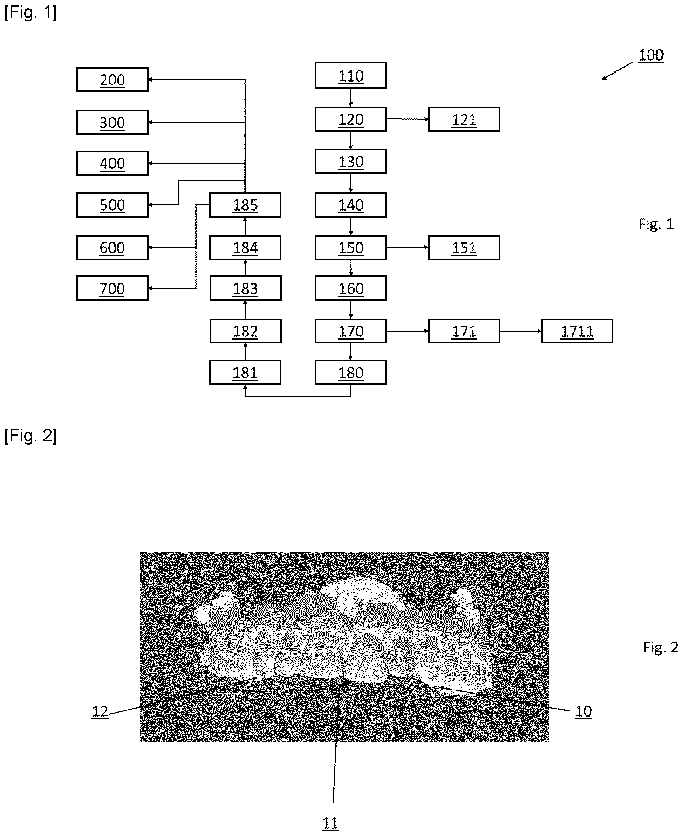

As illustrated in figure 1, the subject application relates to a first computer-implemented method **100**.

In other words, the subject application involves a method that is implemented on a computer.

In the subject application, the first computer-implemented method **100** is specifically intended for estimating one or more scaling factors to map between a 2D digital image of a dentition and a 3D digital model of a dentition and jaw.

As used herein, the term 'scaling factor' refers to a mathematical coefficient used to increase or decrease the magnitude of a particular variable or set of variables. In the context of the first computer-implemented method **100**, one or more scaling factors are estimated to adjust the size, shape, orientation and/or alignment of a 2D digital image of a dentition to match that of a 3D digital model of the dentition and jaw.

Specifically, the estimated one or more scaling factors can be used to project the 2D digital image onto the 3D digital model surface to create a 3D representation from the 2D digital image. Conversely, the 3D digital model can also be projected onto the 2D digital image plane based on the one or more scaling factors.

From an implementation standpoint, these estimated scaling factors may be represented as floating point numeric values. In the simplest case, a single uniform scale ratio may be calculated to be applied to the 3D digital model and 2D digital image globally. More complex implementations may compute multiple localized scaling transformations encoded in matrix form, to enable non-uniform adjustments adapting to region-specific morphometric differences between the 2D digital image and 3D digital model.

The term `2D digital image of a dentition' refers to a two-dimensional representation of a patient's teeth captured digitally. This image provides information on the size and dimensions of the patient's dentition and serves as a reference for dental treatment planning in the first computer-implemented method **100**.

The term '3D digital model of a dentition and jaw' relates to a three-dimensional digital representation of a patient's teeth and jaw. This model, which is more comprehensive than a 2D digital image, provides detailed information about the patient's oral structures, including the shape, size, and spatial relationships of the teeth and jaw. In the first computer-implemented method **100**, this model is used in conjunction with a 2D digital image for dental treatment planning.

In practical terms, estimating scaling factors to map between a 2D digital image of a dentition and a 3D digital model of a dentition and jaw essentially means determining the numerical values needed to adjust the size, shape, orientation and/or alignment of the 2D digital image so that it matches the size, shape, orientation and/or alignment of the 3D digital model, and vice versa.

This is important because the sizes, shapes, orientations and/or alignments of the 2D digital image and the 3D digital model might not naturally match due to differences in the techniques used to create them. For instance, a photograph (2D digital image) of a patient's teeth might appear smaller or larger than a 3D digital model created using a 3D scanner.

The first computer-implemented method **100** resolves this issue by calculating the necessary scaling factors to ensure that the size, shape, orientation and/or alignment of the 2D digital image and the 3D digital model align accurately, facilitating precise analysis and treatment planning in the dental restoration process.

### ► ► First step of the first aspect of the subject: acquiring and aligning a 3D digital model of the dentition and jaw of a patient into a CAD system

As illustrated in figure 1, step **110** of the first computer-implemented method **100** involves acquiring a 3D digital model of the dentition and jaw of a patient, the 3D digital model having been transformed to be centered, aligned with the X, Y, and Z axes of a CAD coordinate system.

As used herein, the term 'CAD coordinate system' refers to a system of coordinates used in Computer-Aided Design (CAD) to locate and orient geometric shapes and models in a defined digital space. CAD is a technology used to create, modify, analyze, or optimize designs digitally. The CAD coordinate system typically includes X, Y, and Z axes, which represent the three dimensions of space.

Furthermore, the 3D digital model is oriented with the frontal view of the dentition facing the positive Y axis of the CAD coordinate system.

As used herein, the term 'frontal view' refers to the perspective of an object or structure as seen directly from the front. It is one of the standard views used in representing 3D objects in 2D spaces, along with the top view and side view. In the context of the first computer-implemented method **100**, the 3D digital model of the dentition and jaw is oriented in the CAD coordinate system with the frontal view of the dentition facing the positive Y axis. This means the front-facing aspect of the dentition and jaw, as if one were looking directly at the patient's smile, is oriented towards the positive direction of the Y axis in the CAD coordinate system.

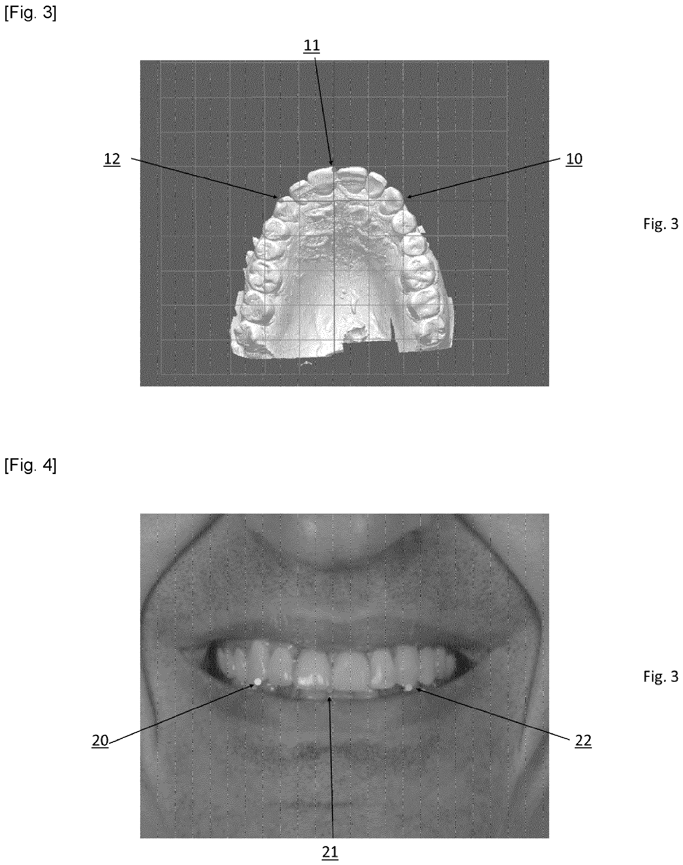

Figure 2 and figure 3 illustrate a 3D digital model of the dentition and jaw of a patient.

This 3D digital model may be obtained through various modalities including intraoral or extra-oral optical scanning, cone beam computed tomography (CBCT), or existing CAD design files from prior procedures. This 3D digital model is not just any representation, but it has undergone transformation to be centered and aligned with the X, Y, and Z axes of the CAD coordinate system.

Additionally, as shown in figure 3, it's oriented in such a way that the frontal view of the dentition is facing the positive Y axis of the CAD coordinate system. This alignment and orientation ensure that the 3D digital model is in the correct position and orientation relative to the 2D digital image.

This step is critical as it allows for a seamless integration of the 2D and 3D data in the CAD system, facilitating the accurate design and planning of dental prosthetic restorations.

### ► ►Second step of the first aspect of the subject: identifying first landmarks on the dentition

Next, in step **120**, the first computer-implemented method **100** identifies, using the 3D digital model, three or more first landmarks on the dentition, wherein the landmarks comprise individual vertices or points on the 3D digital model.

As used herein, the term 'landmark' in the context of a `3D digital model' refers to specific, identifiable points on the 3D digital model that are used as reference points for measurements, alignment, or mapping. They can be natural features, such as the tip of a tooth or the edge of the gumline, or artificial points defined by the user or the software.

Practically, step **120** of the first computer-implemented method **100** involves identifying three or more first landmarks on the dentition using the 3D digital model. This can be done manually or through automated determination using dental image analysis and landmark detection algorithms.

Figure 2 and figure 3 illustrate three first landmarks **10, 11, 12** located on a 3D digital model of the dentition and jaw of a patient.

In an example, the three or more first landmarks **10, 11, 12** on the dentition comprise the cusp tips of the canines, the mesial and distal limits of the incisive edges, inter-canine width, and/or the combination thereof.

These landmarks are individual vertices or points on the 3D digital model.

Identifying these landmarks is an important stage of the process as it provides reference points that can be used for scaling, orienting, and aligning the 2D digital image with the 3D digital model in the CAD system. By selecting individual points on the 3D digital model that accurately represents the patient's dentition, the process ensures a high level of precision in the subsequent stages of the dental prosthetic restoration process.

Technically, these first landmarks **10, 11, 12** on the 3D digital model are represented as sets of 3D coordinates in the X, Y, Z axes of the CAD coordinate system. Each landmark is defined by a unique (X, Y, Z) triplet specifying its exact position in 3 dimensions.

This allows for precise computational comparison and analysis between the 2D and 3D landmark sets to evaluate morphological correspondence.

### ► ►Third step of the first aspect of the subject: calculating first landmark-based geometric morphometrics

In step **130**, the first computer-implemented method **100** calculates first landmark-based geometric morphometrics based on the first landmarks **10, 11, 12**

In other words, in step **130** of the first computer-implemented method **100**, first landmark-based geometric morphometrics are calculated based on the first landmarks **10, 11, 12.**

As used herein, the term `landmark-based geometric morphometrics' refers to the quantitative measurements and numerical analysis of form and shape based on the placement of anatomical landmarks. It involves identifying identical or homologous points (or landmarks) on each object or structure being studied, and then statistically analyzing the coordinates of these landmarks. This analysis provides quantitative data that represents the geometric shape and structure of the objects, allowing for a detailed comparison of their forms.

This calculation involves generating quantitative measurements that describe the shape and arrangement of the first landmarks **10, 11, 12** on the 3D digital model.

These measurements might include the distances between landmarks, the angles formed by lines connecting different landmarks, and other geometric properties.

These first landmark-based geometric morphometrics provide a numerical representation of the patient's dentition as represented in the 3D digital model, which is crucial for the subsequent stages of the CAD process in designing the dental prosthetic restorations.

In an embodiment of step **130**, determining the first landmark-based geometric morphometrics comprises calculating **131** distances between landmarks, computing angles between landmarks, determining the area of polygonal regions defined by multiple landmarks, assessing the curvature of the dental arch at landmark locations, analyzing the relative alignment of landmarks, and/or the combination thereof.

In other words, in an embodiment of step 130, determining the first landmark-based geometric morphometrics involves a range of computational procedures.

These procedures could include calculating distances between landmarks, such as the distance between the cusp tips of the canines.

They could also involve computing angles between landmarks, like the angle between the incisive edges and the cusp tips of the canines.

In addition, the procedures might involve determining the area of polygonal regions defined by multiple landmarks, such as the area enclosed by points on the cusp tips of the canines and the incisive edges.

They could also include assessing the curvature of the dental arch at landmark locations to identify the shape of the arch.

Furthermore, they might involve analyzing the relative alignment of the landmarks, such as the alignment of the cusp tips of the canines with the incisive edges.

Using a combination of these procedures would provide a comprehensive quantitative representation of the patient's dentition, which would help in the design of dental prosthetic restorations using the CAD system.

To further illustrate, the computational procedures described above could leverage specific mathematical formulas.

For instance, calculating distances between landmarks could utilize the Euclidean distance formula, given by d = sqrt[(x2-x1)²+(y2-y1)²+(z2-z1)²], where (x1, y1, z1) and (x2, y2, z2) are the 3D coordinates of two landmarks.

Computing the angle θ (theta) between three landmarks A, B, and C could be done using the following formula: θ = arccos[(AB_x * AC_x + AB_y * AC_y + AB_z * AC_z) / (sqrt(AB_x^2 + AB_y^2 + AB_z^2) * sqrt(AC_x^2 + AC_y^2 + AC_z^2))]
where (AB_x, AB_y, AB_z) and (AC_x, AC_y, AC_z) are the coordinates of vectors AB and AC.

Determining the area of a triangle with vertices at landmarks A, B, and C could involve the formula: A = 0.5 * sqrt[(AB_y * AC_z - AB_z * AC_y)^2 + (AB_z * AC_x - AB_x * AC_z)^2 + (AB_x * AC_y - AB_y * AC_x)^2].

Assessing the mean curvature of the dental arch at a landmark location could be performed using a local curvature formula such as: H = (k1 + k2) / 2,
where k1, k2 are the principal curvatures computed from the surface's fundamental forms.

Analyzing the relative alignment angle θ between 3D vectors A and B could utilize the following formula: θ = arccos[(A_x * B_x + A_y * B_y + A_z * B_z) / (sqrt(A_x^2 + A_y^2 + A_z^2) * sqrt(B_x^2 + B_y^2 + B_z^2))].

Of course, other mathematical formulations are also possible for calculating the second landmark-based geometric morphometrics, and the actual mathematical formulas used could vary depending on specific requirements.

### ► ► Fourth step of the first aspect of the subject: acquiring and aligning a virtual or synthetic 2D digital image of the dentition of a patient into a CAD system

Following this, step **140** of the first computer-implemented method **100** involves acquiring a virtual or synthetic 2D digital image of the dentition of a patient.

As used herein, the term 'virtual 2D digital image' typically refers to an image that is digitally created or manipulated. It could be a photograph that has been digitally scanned or a digital photograph that has been altered or enhanced using software.

Figure 4 illustrates a virtual 2D digital image of the dentition of a patient.

'A synthetic 2D digital image', on the other hand, usually refers to an image that is created from scratch using computer graphics or other digital tools. This could be a computer-generated image that is designed to replicate or represent the dentition of a patient. In the context of this claim, a synthetic image might be used to create an ideal or reference image against which the patient's actual dentition can be compared.

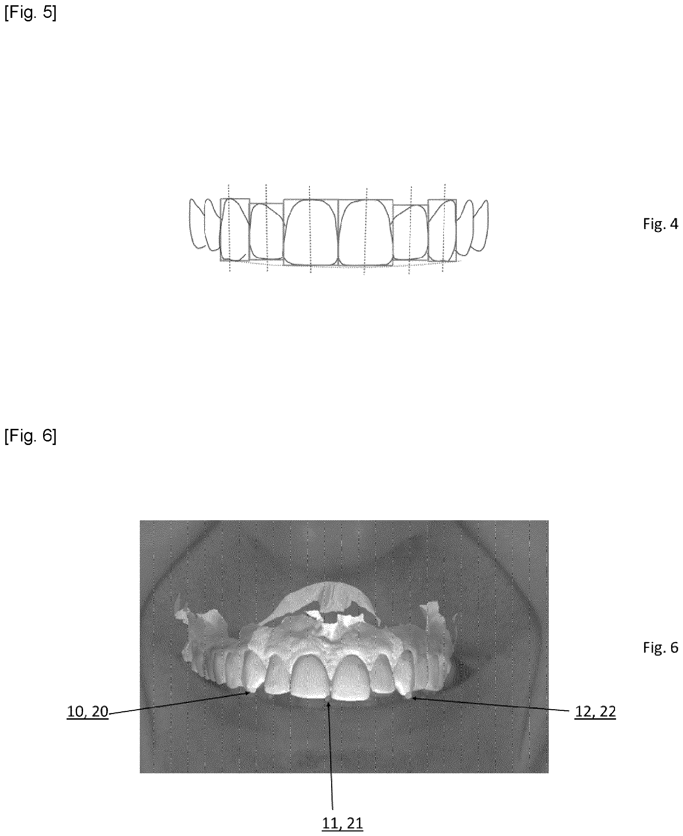

Figure 5 illustrates a synthetic 2D digital image of the dentition of a patient.

In the case of the first computer-implemented method **100**, a virtual or synthetic 2D digital image of a dentition is acquired and used for dental treatment planning.

Furthermore, step **140** of the first computer-implemented method **100** then involves aligning the 2D digital image with the X and Y axes of a CAD coordinate system.

In an example, the 2D digital image is aligned with the origin at the intersection of the X and Y axes of the same CAD coordinate system as the 3D digital model.

In the context of the first computer-implemented method **100**, the 2D digital image of the dentition is aligned with the X and Y axes of the CAD coordinate system, providing a standardized reference for further processing and analysis. Indeed, since the digital image is a 2D representation, only the X and Y axes defining the 2D space need to be considered and/or alignment, as opposed to 3D digital models which utilize all three X, Y and Z axes.

In practice, step **140** of the first computer-implemented method **100** involves aligning the 2D digital image with the X and Y axes of the CAD coordinate system. This means adjusting the orientation of the image so that it fits into the defined 2D space of the CAD system, which is defined by the X and Y axes. This alignment ensures that the image is in the correct position for further processing and analysis in the CAD system. For example, the bottom edge of the image might be aligned with the X-axis, and the left edge with the Y-axis. This step is crucial for maintaining consistency and accuracy in the subsequent stages of the dental prosthetic restoration process.

The alignment process in step **140** may typically utilize image processing algorithms and software tools to analyze the orientation of the 2D digital image and make adjustments to straighten it and fit it to the X and Y axes. Common techniques include detecting edge features, analyzing pixel patterns, and applying geometric transformations like rotation and translation to properly position the image. The algorithms can process details like skew and distortion that may be present in the captured photo to standardize the perspective.

With the 2D digital image now aligned to the CAD coordinate system's X and Y axes, subsequent steps in the dental prosthetic restoration workflow can rely on this standardized reference frame. For example, the aligned image can be used to take accurate measurements of tooth and gum dimensions or to design prosthetic models that will properly fit the patient's mouth anatomy. Standardizing on the same coordinate system ensures that all data such as scan files, measurements, and 3D printed components share the same frame of reference for proper integration.

### ► ► Fifth step of the first aspect of the subject: identifying second landmarks on the dentition

Next, in step **150**, the first computer-implemented method **100** identifies, using the 2D digital image, three or more second landmarks on the dentition, wherein the landmarks comprise individual points on the 2D digital image, and are selected to correspond to the same anatomical locations as the first landmarks **10, 11, 12** determined in the 3D digital model.

While conceivably possible to use fewer than three landmarks in certain use cases, at least three separate identifiable points of reference are required to enable the necessary alignment, scaling, orientation and computational analysis of the 2D digital image for precision and accuracy in subsequent dental prosthetic design procedures.

As used herein, the term 'landmark' in the context of a '2D digital image of a dentition' refers to specific, identifiable points on the image that are used as reference points for measurements, alignment, or mapping. They can be natural features, such as the tip of a tooth or the edge of the gumline, or artificial points defined by the user or the software.

In effect, step **150** of the first computer-implemented method **100** involves identifying three or more second landmarks on the dentition using the 2D digital image. This can be done manually or through automated determination using dental image analysis and landmark detection algorithms. These landmarks are specific points on the 2D digital image that correspond to distinct and identifiable features on the dentition.

Figure 4 illustrates three second landmarks **20, 21, 22** located on a 2D digital image of the dentition of a patient.

In an example, the three or more second landmarks **20, 21, 22** on the dentition comprise the cusp tips of the canines, the mesial and distal limits of the incisive edges, inter-canine width, and/or the combination thereof.

These landmarks are individual vertices or points on the 2D digital image that correspond to the same anatomical locations as the first landmarks **10, 11, 12** determined in the 3D digital model.

For instance, if a particular landmark in the 3D digital model is the tip of a canine tooth, the corresponding second landmark in the 2D digital image would be the corresponding point representing the tip of the same canine tooth.

This step is crucial as it ensures that the 3D digital model and the 2D digital image are in sync with each other, enabling an accurate representation of the patient's dentition in the CAD system for prosthetic restoration design.

### ► ►Sixth step of the first aspect of the subject: calculating second landmark-based geometric morphometrics

In step **160**, the first computer-implemented method **100** calculates second landmark-based geometric morphometrics based on the second landmarks **20, 21, 22.**

Essentially, in step **160**, the first computer-implemented method **100** involves computing second landmark-based geometric morphometrics based on the second landmarks **20, 21, 22.** This means that the system calculates quantitative measurements that describe the shape and arrangement of the second landmarks **20, 21, 22** on the 2D digital image. These measurements might include, for example, the distances between landmarks, the angles formed by lines connecting different landmarks, and other geometric properties similar to the first landmark-based geometric morphometrics calculated from the 3D digital model. These second landmark-based geometric morphometrics provide a numerical representation of the patient's dentition, which can be utilized in subsequent stages of the CAD process for designing dental prosthetic restorations.

In an embodiment of step **160**, determining the second landmark-based geometric morphometrics comprises calculating **161** distances between landmarks, computing angles between landmarks, determining the area of polygonal regions defined by multiple landmarks, assessing the curvature of the dental arch at landmark locations, analyzing the relative alignment of landmarks, and/or the combination thereof.

In other words, in an embodiment of step **160**, determining the second landmark-based geometric morphometrics involves a range of computational procedures.

These procedures could include calculating distances between landmarks, such as the distance between the cusp tips of the canines.

They could also involve computing angles between landmarks, like the angle between the incisive edges and the cusp tips of the canines.

In addition, the procedures might involve determining the area of polygonal regions defined by multiple landmarks, such as the area enclosed by points on the cusp tips of the canines and the incisive edges.

They could also include assessing the curvature of the dental arch at landmark locations to identify the shape of the arch.

Furthermore, they might involve analyzing the relative alignment of the landmarks, such as the alignment of the cusp tips of the canines with the incisive edges.

Using a combination of these procedures would provide a comprehensive quantitative representation of the patient's dentition, which would help in the design of dental prosthetic restorations using the CAD system.

Similar mathematical formulations as described above with respect to the first landmark-based geometric morphometrics are also possible for calculating the second landmark-based geometric morphometrics, and the actual mathematical formulas used could vary depending on specific requirements.

### ► ►Seventh step of the first aspect of the subject: estimating one or more scaling factors

Finally, the first computer-implemented method **100** estimates **170** one or more scaling factors based on the comparison **171** of the first and second landmark-based geometric morphometrics. This concludes the steps of the first computer-implemented method **100.**

In practice, the final step of the first computer-implemented method **100** includes estimating one or more scaling factors. These scaling factors are calculated based on the comparison **171** of the first and second landmark-based geometric morphometrics.

This comparison essentially involves analyzing the numerical measurements derived from the landmarks on the 2D digital image and the 3D digital model.

The one or more scaling factors serve to align the size, shape, orientation and/or alignment of the landmarks on the 3D digital model with the corresponding landmarks on the 2D digital image.

This estimation completes the steps of the first computer-implemented method **100** and aids in creating a more accurate and precise 3D representation of the patient's dentition in the CAD system for designing the dental prosthetic restorations.

In an embodiment of step **171**, comparing the first and second landmark-based geometric morphometrics comprises calculating **1711** one or more scaling factors that minimizes the differences between the,
- calculated distances between landmarks,
- computed angles between landmarks,
- determined areas of polygonal regions defined by multiple landmarks,
- assessed curvatures of the dental arch at landmark locations,
- analyzed relative alignments of landmarks, and
any combination thereof.

In practice, an embodiment of step **171** involves comparing the first and second landmark-based geometric morphometrics.

This comparison entails calculating **1711** one or more scaling factors that minimize the discrepancies between various measurements and assessments.

For instance, these could include the calculated distances between landmarks, such as the distance between the cusp tips of the canines in both the 2D digital image and the 3D digital model.

They could also involve the computed angles between landmarks, the determined areas of polygonal regions defined by multiple landmarks, and the assessed curvatures of the dental arch at landmark locations.

The comparison also includes the analyzed relative alignments of landmarks.

By minimizing the differences between these measurements and assessments in the 2D digital image and the 3D digital model, the one or more scaling factors help ensure that the 3D digital model accurately represents the patient's dentition as shown in the 2D digital image.

This process aids in the precise design of dental prosthetic restorations using the CAD system.

In a particular implementation of the embodiment of step **171**, the calculation of appropriate scaling factor(s) to align the 2D and 3D dental images relies on mathematical comparison of the geometric morphometrics determined for each dataset. Possible approaches include:
- statistical analysis to derive a uniform scaling factor based on computing ratios and means of corresponding landmark distances and measurements,
- optimization algorithms designed to numerically minimize the difference in morphometrics by adjusting scaling iteratively, and
- machine learning techniques leveraging training data to predict suitable scaling transformations between 2D and 3D pairs.

The output is one or more scaling factors that can then programmatically adjust the 3D model to maximize morphometric correspondence with the 2D digital image based on the anatomical landmarks.

In particular, the step **1711** of calculating the one or more scaling factors may comprises one or more of the following methods:
- calculating an average ratio of corresponding landmark distances in the 2D digital image and the 3D digital model,
- applying a least squares method to minimize the sum of the squares of the differences between the observed and estimated values of the geometric morphometrics,
- training a machine learning model on a dataset of 2D and 3D images with known scaling factors to predict the one or more scaling factors for a new pair of 2D and 3D images,
- using iterative refinement to start with an initial estimate for the one or more scaling factors, then refine this estimate by comparing the geometric morphometrics of the scaled 3D digital model and the 2D digital image until the difference falls below a certain threshold, and
- implementing an optimization algorithm such as gradient descent to find the one or more scaling factors that minimize the difference between the geometric morphometrics of the 2D digital image and the 3D digital model.

In that particular implementation of the embodiment of step **171**, the step **1711** of calculating one or more scaling factors encompasses several possible methods.

For instance, it could involve calculating an average ratio of corresponding landmark distances in the 2D digital image and the 3D digital model. This could be expressed as Σ(d2D_i/d3D_i)/n, where d2D_i and d3D_i are the ith corresponding landmark distances in the 2D digital image and the 3D digital model, respectively, and n is the total number of corresponding landmarks. This could help in establishing a uniform scaling factor for all the landmarks.

Also, it could involve applying a least squares method to minimize the sum of the squares of the differences between the observed and estimated values of the geometric morphometrics. This could be expressed as Σ(observed_i - estimated_i)².

Alternatively, it could involve training a machine learning model on a dataset of 2D and 3D images with known scaling factors. The trained model could then be used to predict the one or more scaling factors for a new pair of 2D and 3D images.

The method could also involve using iterative refinement. Here, the process would start with an initial estimate for one or more the scaling factors. This estimate would then be refined by comparing the geometric morphometrics of the scaled 3D digital model and the 2D digital image until the difference, expressed as |Σ(observed_i - estimated_i)|, falls below a certain threshold.

Lastly, it could involve implementing an optimization algorithm, such as gradient descent. This algorithm would find the one or more scaling factors that minimize the difference between the geometric morphometrics of the 2D digital image and the 3D digital model. Gradient descent could be expressed as θ = θ - α * ∇J(θ), where θ is the scaling factor, α is the learning rate, and J(θ) is the cost function representing the difference between the geometric morphometrics of the 2D digital image and the 3D digital model.

These methods would allow for a precise scaling factor, ensuring an accurate representation of the patient's dentition in the 3D digital model, aiding in the design of dental prosthetic restorations.

### ►►►A first embodiment of the first aspect of the subject application: aligning the 3D digital model to the 2D digital image

In a first embodiment of the subject application, the first computer-implemented method **100** further comprises,
- using **180** the one or more scaling factors to adjust the size, shape, orientation and/or alignment of the 3D digital model to correspond with the size, shape, orientation and/or alignment of the 2D digital image, thereby generating an adjusted 3D digital model,
- positioning **181** the adjusted 3D digital model to align its landmarks with the corresponding landmarks of the 2D digital image, thereby generating a positioned adjusted 3D digital model, and
- superimposing **182** the positioned adjusted 3D digital model over the 2D digital image.

In other words, in a first embodiment of the subject application, the first computer-implemented method **100** adds more procedures to the first aspect of the subject application.

The first computer-implemented method **100** includes using **180** the one or more scaling factors, calculated with the first aspect of the subject application, to adjust the size, shape, orientation and/or alignment of the 3D digital model to match the size, shape, orientation and/or alignment of the 2D digital image, thus generating an adjusted 3D digital model.

For instance, if the one or more scaling factors indicate that the 3D digital model is larger than the 2D digital image, the method would reduce the size, shape, orientation and/or alignment of the 3D digital model to match that of the 2D digital image.

Following this, the first computer-implemented method **100** includes positioning **181** the adjusted 3D digital model so its first landmarks align with the corresponding second landmarks of the 2D digital image.

This step generates a positioned and adjusted 3D digital model. For example, the cusp tips of the canines in the 3D digital model would be aligned with the cusp tips of the canines in the 2D digital image.

Figure 6 illustrates a result of the above-mentioned positioning.

Finally, the first computer-implemented method **100** includes superimposing **182** the positioned adjusted 3D digital model over the 2D digital image.

This overlay provides a visual confirmation that the 3D digital model accurately represents the patient's dentition as shown in the 2D digital image.

These steps ensure that the final 3D digital model used in the CAD system for designing dental prosthetic restorations is as accurate as possible.

In an implementation of the first embodiment of the subject application, the first computer-implemented method **100** further comprises,
- deriving **183** a dental arch line on the positioned adjusted 3D digital model based on the first landmarks **10, 11, 12** and/or the first landmark-based geometric morphometrics,
- deriving **184** a dental arch line on the 2D digital image based on the second landmarks **20, 21, 22** and/or the second landmark-based geometric morphometrics, and
- adjusting **185** the derived dental arch line on the 2D digital image to match the derived dental arch line from the positioned adjusted 3D digital model.

In practice, in an implementation of the first embodiment of the subject application, the first computer-implemented method **100** involves additional steps that focus on dental arch lines.

This method includes deriving **183** a dental arch line on the positioned adjusted 3D digital model based on the first landmarks **10, 11, 12** and/or the first landmark-based geometric morphometrics.

For example, the dental arch line can be derived by connecting the landmarks denoting the cusps or tips of the teeth in the 3D digital model.

The method also involves deriving **184** a dental arch line on the 2D digital image based on the second landmarks **20, 21, 22** and/or the second landmark-based geometric morphometrics.

As with the positioned adjusted 3D digital model, the dental arch line can be derived by connecting the landmarks denoting the cusps or tips of the teeth in the 2D digital image.

Finally, the method includes adjusting **185** the derived dental arch line on the 2D digital image to match the derived dental arch line from the positioned adjusted 3D digital model.

This adjustment ensures that the arch lines in both the 2D digital image and the 3D digital model are as similar as possible, further improving the accuracy of the 3D digital model used in the CAD system for designing dental prosthetic restorations.

### ►First variation of the first aspect of the subject application: a second computer-implemented method for digitally preparing teeth

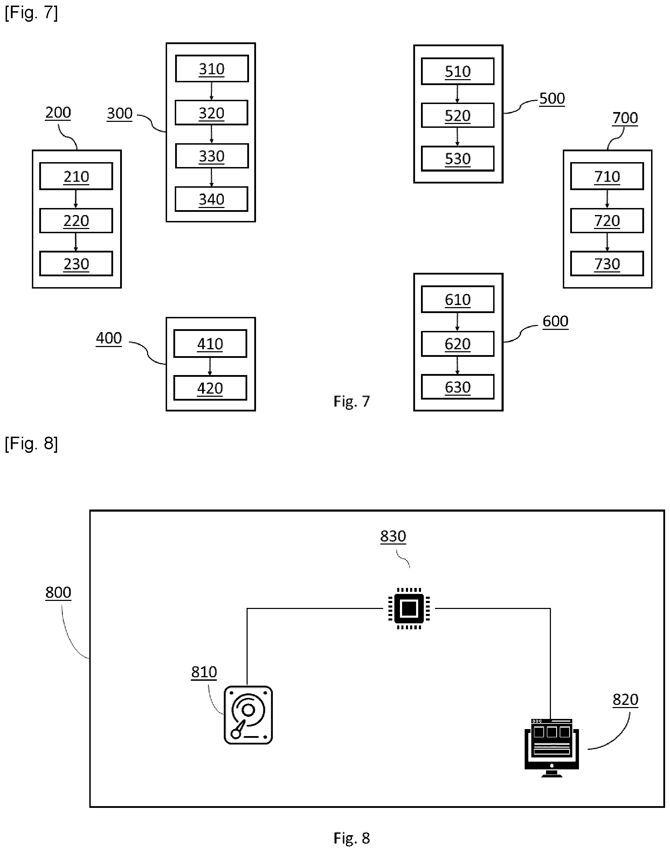

As illustrated in figure 1 and figure 7, the subject application also relates to a second computer-implemented method **200.**

In other words, the subject application involves a method that is implemented on a computer.

In the subject application, the second computer-implemented method **200** is specifically intended for digitally preparing natural teeth for prosthetic dental restorations.

As used herein, the term 'digitally preparing natural teeth' refers to using computer software techniques to virtually adjust the surface and shape of a 3D digital model representing the patient's natural teeth.

This is done to sculpt the digital teeth in areas that will receive dental prosthetic restorations, ensuring a perfect fit. The digital preparation provides spatial accommodation for prosthetic elements.

It may involve techniques such as digital sculpting, mesh editing, boolean operations, and cutting/slicing tools to manipulate the 3D model.

First, step **210** of the second computer-implemented method **200** involves acquiring **210** a first positioned adjusted 3D digital model, as generated by the first computer-implemented method **100**, to represent one or more unprepared natural teeth of a patient along with the associated gum.

Here, the term 'unprepared natural teeth' refers to the patient's original dental anatomy before any modifications have been made to accommodate future dental prosthetics.

Next, in step **220**, the second computer-implemented method **200** acquires a second positioned adjusted 3D digital model, as generated by the first computer-implemented method **100**, that digitally represents proposed changes and/or improvements to the natural teeth, thereby illustrating an anticipated final outcome of the prosthetic dental restoration.

Here, the term 'final outcome' refers to the expected end result after the placement of the dental prosthetics on the digitally prepared natural teeth.

Finally, the second computer-implemented method **200** uses **230** a Computer-Aided Design system, CAD, to digitally prepare the natural teeth for prosthetic dental restorations based on the first and second positioned adjusted 3D digital models, thereby creating a 3D digital dental model.

Specifically, the CAD system allows performing digital sculpting and other digital adjustment techniques on the 3D model of the natural teeth to create space and customize areas receiving dental prosthetics. This virtual preparation in the CAD environment is analogous to anatomical preparation in real-life procedures but is non-invasive and allows additional simulation and optimization.

### ►Second variation of the first aspect of the subject application: a third computer-implemented method for generating toolpaths

As illustrated in figure 1 and figure 7, the subject application also relates to a third computer-implemented method **300**.

In other words, the subject application involves a method that is implemented on a computer.

In the subject application, the third computer-implemented method **300** is specifically intended for generating toolpaths to prepare natural teeth for prosthetic dental restorations.

In real-life dental procedures, standard instruments used to physically prepare teeth for prosthetic restorations include but are not limited to: highspeed dental handpieces, slow-speed hand instruments, air-abrasion devices, laser ablation devices, and mesio-occluso-distal (MOD) preparation burs.

The specific tools used depend on the individual case, location of preparations needed, and dentist's preferences. These physical preparations allow properly adjusting the shape and size of the patient's natural teeth to facilitate subsequent placement of prosthetic restorations.

The first step **310** of the third computer-implemented method **300** involves acquiring a first positioned adjusted 3D digital model, as generated by the first computer-implemented method **100**, to represent one or more unprepared natural teeth of a patient along with the associated gum.

Next, in step **320**, the third computer-implemented method **300** acquires a second positioned adjusted 3D digital model, as generated by the second computer-implemented method **200**, that digitally represents the prepared natural teeth of the patient along with the associated gum, wherein the prepared natural teeth correspond to the unprepared natural teeth whose surface has been prepared to coincide with one or more offset intrusion zones to increase its perceptibility, each offset intrusion zone representing an area where an inward offset penetrates the first positioned adjusted 3D digital model.

Then, in step **330**, the third computer-implemented method **300** generates **330** tool paths based on the first and second positioned adjusted 3D digital models to prepare the natural teeth for prosthetic dental restorations.

The toolpaths in step **330** may be generated using digital dental modeling tools and CAD/CAM software. These tools allow virtually sculpting the 3D digital models representing the prepared and unprepared teeth to calculate optimal trajectories for surgical instruments to carry out the necessary dental preparations.

For example, toolpaths can guide a robotic drill along the desired shape and depth when physically adjusting the patient's teeth prior to taking impressions or placing permanent prosthetic restorations.

Finally, the third computer-implemented method **300** provides **340** instructions or parameters to a Computer-Aided Manufacturing, CAM, system for preparing the natural teeth based on the generated toolpaths.

### ►Third variation of the first aspect of the subject application: a fourth computer-implemented method for designing dental prosthetics

As illustrated in figure 1 and figure 7, the subject application also relates to a fourth computer-implemented method **400.**

In other words, the subject application involves a method that is implemented on a computer.

In the subject application, the fourth computer-implemented method **400** is specifically intended for designing one or more dental prosthetics.

The first step **410** of the fourth computer-implemented method **400** involves using, by a design system or device, a positioned adjusted 3D digital model, as generated by the second computer-implemented method **200**, to design the dental prosthetics such that they fit within the patient's dentition.

Specifically, the design system or device may use CAD software and dental restoration design techniques to meticulously map the surface of the positioned adjusted 3D digital model representing the prepared teeth.

This allows creating prosthetic components like copings and crowns that perfectly match the unique anatomy of the patient's dentition.

Finally, the fourth computer-implemented method **400** provides **420** instructions or parameters to a manufacturing system or device for producing the one or more prosthetics based on the design.

The instructions or parameters provided at step **420** could include specific subclassification codes, material choice, color coding, articulation parameters, occlusion mapping, and finish line trajectories compatible with the target manufacturing system.

Additionally, some examples of manufacturing systems that could receive these instructions or parameters are:
- Computer Numerical Control (CNC) milling machines to freshen denture surfaces,
- 3D printers to produce resin crowns or bridges,
- Laser welding systems to join metal frameworks, and
- Electrical Discharge Machining (EDM) machining to create ceramic copings.

### ►Fourth variation of the first aspect of the subject application: a fifth computer-implemented method for generating customized dental implant surgery plans

As illustrated in figure 1 and figure 7, the subject application also relates to a fifth computer-implemented method **500.**

In other words, the subject application involves a method that is implemented on a computer.

In the subject application, the fifth computer-implemented method **500** is specifically intended for generating customized dental implant surgery plans.

More specifically, planning the dental implant surgeries involves analyzing the positioned 3D digital model to simulate implantation based on the patient's bone structure and anatomy. By assessing density, volume, and shape, the design system determines the optimum number, size, positioning, and orientation of implants to support long-term restorative needs.

The first step **510** of the fifth computer-implemented method **500** involves using, by a design system or device, a positioned adjusted 3D digital model, as generated by the first computer-implemented method **100**, to plan the dental implant surgeries.

Next, in step **520**, the fifth computer-implemented method **500** determines the appropriate size and position of dental implants based on this plan to ensure optimal placement within the patient's jaw.

Dental implants themselves are prosthetic components typically made of biocompatible titanium or ceramic materials like zirconia. They are surgically inserted into the jawbone as artificial tooth root substitutes that integrate with the patient's own bone over 3-6 months through a process known as osseointegration.

This provides a stable foundation to anchor restorative solutions like crowns, bridges, or dentures.

Finally, the fifth computer-implemented method **500** provides implant placement instructions or guidelines for a dental professional based on the plan.

Precisely following the computer-generated implant placement instructions helps ensure surgical success and patient satisfaction. Optimal positioning coordinates guide gentle drilling sequences to prepare sites for each planned implant.

This maximizes contact with intact bone, promoting faster healing and better integration while minimizing surgical complications. Clear guidelines allow accurate translation of the digital plan to reality.

In practice, the custom implant placement instructions are computer-generated by the planning software based on analysis of the 3D scans and simulation of optimal positioning.

For each planned implant, these contain detailed specifications such as exact 3D coordinate location, drill depth, angulation parameters, and product selection.

For instance, the guidelines for a mandibular second left molar implant could state:
- X=47.32mm Y=-36.41mm Z=-16.54mm
- Drill depth=10.5mm
- Tilted 35 degrees distal from frontal plane
- Recommended implant: Straumann Bone Level Ø3.3mm RC L10mm.

### ► Fifth variation of the first aspect of the subject application: a sixth computer-implemented method for creating orthodontic treatment plans

As illustrated in figure 1 and figure 7, the subject application also relates to a sixth computer-implemented method **600**.

In other words, the subject application involves a method that is implemented on a computer.

In the subject application, the sixth computer-implemented method **600** is specifically intended for creating orthodontic treatment plans.

As generally known in the art of orthodontics, orthodontic treatment plans outline the sequenced movement of teeth over a course of care using dental appliances to improve alignment and occlusion.

Common orthodontic appliances include brackets, archwires, elastic bands, and retainers customized for the patient.

The first step **610** of the sixth computer-implemented method **600** involves using **610**, by a design system or device, a positioned adjusted 3D digital model, as generated by the first computer-implemented method **100**, to create the treatment plans.

Next, in step **620**, the sixth computer-implemented method **600** designs and sizes **620** orthodontic appliances based on the treatment plans to fit within the patient's dentition.

Finally, the sixth computer-implemented method **600** provides specifications or parameters to a manufacturing system or device for producing the orthodontic appliances based on the design.

As with dental prosthetics, the manufacturing systems receive digital instructions to produce the custom appliances.

### ►Sixth variation of the first aspect of the subject application: a seventh computer-implemented method for generating forensic dental analysis data

As illustrated in figure 1 and figure 7, the subject application also relates to a seventh computer-implemented method **700**.

In other words, the subject application involves a method that is implemented on a computer.

In the subject application, the seventh computer-implemented method **700** is specifically intended for generating forensic dental analysis data.

For instance, the applied forensic techniques involve comparative analysis of dental impressions, radiographs, and 3D digital models from suspects and evidence to facilitate human identification and investigation of biting injuries.

The first step **710** of the seventh computer-implemented method **700** involves using, by a design system or device, the positioned adjusted 3D digital model, as generated by the first computer-implemented method **100**, for the applications.

In particular, the positioned adjusted 3D digital model accurately captures dental arch shapes to simulate bite marks for comparison against evidence collected from victims and crime scenes.

Next, in step **720**, the seventh computer-implemented method **700** generates data for identifying individuals and analyzing bite marks based on the positioned adjusted 3D digital model.

In an example, the identification data digitally generated includes quantifiable tooth geometry, unique surface wear patterns, spacing, rotations, and other features creating a distinctive "dental fingerprint" signature for subjects.

Finally, the seventh computer-implemented method **700** provides **730** analysis data or identification markers for forensic professionals to support their investigations.

Examples of precise metric analysis markers supplied to forensic teams may include inter-canine and arch width distances, tooth angulations, contour curvatures, and occlusion bite alignments.

### ►Second aspect of the subject application: a computer-readable medium

The subject application also relates to a computer-readable medium having stored thereon computer instructions which when executed, by a processor, perform one or more of the first, second, third, fourth, fifth, sixth and seventh computer-implemented methods **100**, **200**, **300**, **400**, **500**, **600, 700** as described above.

Specifically, the software program contains complex computational algorithms encoded to execute various functions including 3D dental image processing, tooth movement simulations, generating personalized 3D digital models, and forensic bite mark analysis.

The computer-readable medium supporting the software instructions can include internal and external memory hardware devices such as hard drives, USB drives, CD-ROM discs, or random access memory containing the programmed code for these computational methods.

An advanced processor with specialized graphics and math processing capabilities may be required to computationally execute the complex 3D modeling and real-time simulations involved in these techniques.

### ►Third aspect of the subject application: a CAD System

As illustrated in figure 8, the subject application also relates to a 3D Computer-Aided Design, CAD, system **800**, which comprises several components.

The first component is one or more memories **810**. More specifically, the digital storage memories may include large capacity hard drives for databases and working memory chips providing fast access speed for real-time computation.

These memories **810** are responsible for storing one or more 2D digital images of dentitions of one or more patients, one or more 3D digital models of dentitions and jaws of the one or more patients and executable instructions for performing one or more of the first, second, third, fourth, fifth, sixth and seventh computer-implemented methods **100**, **200**, **300**, **400, 500, 600, 700.**

The CAD system **800** also includes one or more user interfaces **820**. The user interfaces may incorporate high-resolution touchscreen monitors, keyboards, and pointing devices to enable visualization of 3D models and user interaction with the software.

The last main component is one or more processors **830**. Advanced graphics processing units and math coprocessors may be embedded in the processors to perform the complex floating-point calculations needed for accurate real-time dental imaging and simulations.

The one or more processors **830** are coupled with the one or more memories **810** and the one or more user interfaces **820**, and they're configured for executing the executable instructions stored in the one or more memories **810.**

Finally, an internal bus network may interconnect the hardware components and embedded firmware to create an integrated CAD system capable of executing all the described computational methods.

### ►Fourth aspect of the subject application: an Apparatus

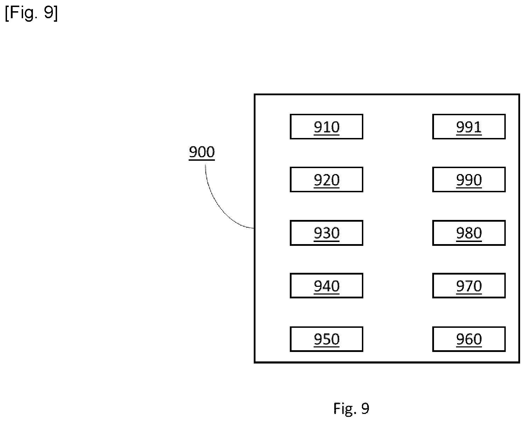

As illustrated in figure 9, the subject application also relates to an apparatus **900.**

In the subject application, the apparatus **900** is specifically designed and built for estimating one or more scaling factors to map between a 2D digital image of a dentition and a 3D digital model of a dentition and jaw.

In particular, the apparatus **900** comprises a processor-based computing system **910** configured to acquire a 3D digital model of the dentition and jaw of the patient, and a virtual or synthetic 2D digital image of the dentition of a patient that accurately represents the size and dimensions of the patient's dentition, as already explained above.

Then, the apparatus **900** comprises a Processor-driven CAD coordinate system **920** wherein the 2D digital image is aligned with the X and Y axes, and the 3D digital model is centered and aligned with the X, Y, and Z axes, with the frontal view of the dentition facing the positive Y axis, as already explained above.

Further, the apparatus **900** comprises an image processor **930** configured to determine three or more first landmarks **10, 11, 12** on the dentition in the 3D digital model three or more second landmarks **20, 21, 22** on the dentition in the 2D digital image, wherein the second landmarks **20, 21, 22**, are selected to correspond to the same anatomical locations as the first landmarks **10, 11, 12** as already explained above.

Still further, the apparatus **900** comprises a processor-operated geometric morphometric calculator **940** designed to calculate first and second landmark-based geometric morphometrics based on the first landmarks **10, 11, 12** and second landmarks **20, 21, 22** respectively, as already explained above.

### ►An embodiment of the processor-operated geometric morphometric calculator

In an embodiment of the processor-operated geometric morphometric calculator **940**, it is further configured to perform operations including calculating distances between landmarks, computing angles between landmarks, determining the area of polygonal regions defined by multiple landmarks, assessing the curvature of the dental arch at landmark locations, analyzing the relative alignment of landmarks, and combinations thereof, as already explained above.

Finally, the apparatus **900** comprises a processor-run scaling estimator **950** configured to estimate one or more scaling factors based on the comparison of the first and second landmark-based geometric morphometrics, as already explained above.

### ►A first embodiment of the processor-run scaling estimator

In a first embodiment of the processor-run scaling estimator **950**, it is further configured to compare the first and second landmark-based geometric morphometrics by calculating one or more scaling factors that minimizes the differences between the,
- calculated distances between landmarks,
- computed angles between landmarks,
- determined areas of polygonal regions defined by multiple landmarks,
- assessed curvatures of the dental arch at landmark locations,
- analyzed relative alignments of landmarks, and
any combination thereof, as already explained above.

### ►A second embodiment of the processor-run scaling estimator

In a second embodiment of the processor-run scaling estimator **950**, it is further configured to calculate the one or more scaling factors using one or more of the following methods, as already explained above:
- calculating an average ratio of corresponding landmark distances in the 2D digital image and the 3D digital model,
- applying a least squares method to minimize the sum of the squares of the differences between the observed and estimated values of the geometric morphometrics,
- training a machine learning model on a dataset of 2D and 3D images with known scaling factors to predict the one or more scaling factors for a new pair of 2D and 3D images,

- using iterative refinement to start with an initial estimate for the one or more scaling factors, then refine this estimate by comparing the geometric morphometrics of the scaled 3D digital model and the 2D digital image until the difference falls below a certain threshold, and
- implementing an optimization algorithm such as gradient descent to find the one or more scaling factors that minimize the difference between the geometric morphometrics of the 2D digital image and the 3D digital model.

### ►An embodiment of fourth aspect of the subject application

In an embodiment of the fourth aspect of the subject application, the apparatus **900** further comprises a processor-model adjuster **960** configured to use the one or more scaling factors to adjust the size, shape, orientation and/or alignment of the 3D digital model to correspond with the size, shape, orientation and/or alignment of the 2D digital image, thereby generating an adjusted 3D digital model, as already explained above.

Then, the apparatus **900** further comprises a processor-controlled positioner **970** configured to align the landmarks of the adjusted 3D digital model with the corresponding landmarks of the 2D digital image, thereby generating a positioned adjusted 3D digital model, as already explained above.

Finally, the apparatus **900** further comprises a processor-guided superimposer **980** configured to overlay the positioned adjusted 3D digital model over the 2D digital image, as already explained above.

### ►►A variation of the embodiment of the fourth aspect of the subject application

In the variation of the embodiment of the fourth aspect of the subject application, the apparatus **900** further comprises a processor-operated dental arch line deriver **990** configured to derive a dental arch line on the 2D digital image based on the second landmarks **20, 21, 22** and/or the second landmark-based geometric morphometrics, as already explained above.

Also, the processor-operated dental arch line deriver **990** is further configured to derive a dental arch line on the positioned adjusted 3D digital model based on the first landmarks **10, 11, 12** and/or the first landmark-based geometric morphometrics, as already explained above.

Finally, the apparatus **900** further comprises a processor-run arch line adjuster **991** configured to adjust the derived dental arch line on the 2D digital image to match the derived dental arch line from the positioned adjusted 3D digital model, as already explained above.

The description of the subject application has been presented for purposes of illustration and description but is not intended to be exhaustive or limited to the application in the form disclosed.

The embodiments were chosen and described to better explain the principles of the application and the practical application, and to enable the skilled person to understand the application for various embodiments with various modifications as are suited to the particular use contemplated.

For instance, the person skilled in the art of the invention can understand that, either the sequence of steps 110, 120 and 130 may be completed before starting the sequence of steps 140, 150 and 160, or the sequence of steps 140, 150 and 160 may be completed before starting the sequence of steps 110, 120 and 130. Hence, the order between the two groups of sequence of steps is reversible, but the internal order within each group should be maintained.

## Claims

1. A first computer-implemented method **(100)** specifically intended for estimating one or more scaling factors to map between a 2D digital image of a dentition and a 3D digital model of a dentition and jaw, the first computer-implemented method **(100)** comprising the steps of,
- acquiring **(110)** a 3D digital model of the dentition and jaw of the patient, the 3D digital model having been transformed to be centered, aligned with the X, Y, and Z axes of a CAD coordinate system, and oriented with the frontal view of the dentition facing the positive Y axis of the CAD coordinate system,
- identifying **(120)**, using the 3D digital model, three or more first landmarks **(10, 11, 12)** on the dentition, wherein the landmarks comprise individual vertices or points on the 3D digital model,
- calculating **(130)** first landmark-based geometric morphometrics based on the first landmarks **(10, 11, 12),**
- acquiring **(140)** a virtual or synthetic 2D digital image of the dentition of a patient, the 2D digital image representing the size and dimensions of the patient's dentition, and aligning the 2D digital image with the X and Y axes of the same CAD coordinate system as the 3D digital image,
- identifying **(150)**, using the 2D digital image, three or more second landmarks **(20, 21, 22)** on the dentition, wherein the landmarks comprise individual points on the 2D digital image, and are selected to correspond to the same anatomical locations as the first landmarks **(10, 11, 12)** determined in the 3D digital model,
- calculating **(160)** second landmark-based geometric morphometrics based on the second landmarks **(20, 21, 22)**,
- estimating **(170)** one or more scaling factors based on the comparison **(171)** of the first and second landmark-based geometric morphometrics.

2. The first computer-implemented method **(100)**of claim 1, wherein the first landmarks **(10, 11, 12)** and/or second landmarks **(20, 21, 22)** comprise the cusp tips of the canines, the mesial and distal limits of the incisive edges, inter-canine width, and/or the combination thereof.

3. The first computer-implemented method **(100)** of any one of claims 1 to 2, wherein identifying **(120**, **150)**, the first and second landmark-based geometric morphometrics comprises calculating **(131**, **161)**, distances between landmarks, computing angles between landmarks, determining the area of polygonal regions defined by multiple landmarks, assessing the curvature of the dental arch at landmark locations, analyzing the relative alignment of landmarks, and/or the combination thereof.

4. The first computer-implemented method **(100)** of claim 3, wherein comparing **(171)** the first and second landmark-based geometric morphometrics comprises calculating **(1711)** one or more scaling factors that minimizes the differences between the,
- calculated distances between landmarks,
- computed angles between landmarks,
- determined areas of polygonal regions defined by multiple landmarks,
- assessed curvatures of the dental arch at landmark locations,
- analyzed relative alignments of landmarks, and
any combination thereof.

5. The first computer-implemented method **(100)** of claim 4, wherein calculating **(1711)** the one or more scaling factors comprises one or more of the following methods:
- calculating an average ratio of corresponding landmark distances in the 2D digital image and the 3D digital model,
- applying a least squares method to minimize the sum of the squares of the differences between the observed and estimated values of the geometric morphometrics,
- training a machine learning model on a dataset of 2D and 3D images with known scaling factors to predict the one or more scaling factors for a new pair of 2D and 3D images,
- using iterative refinement to start with an initial estimate for the one or more scaling factors, then refine this estimate by comparing the geometric morphometrics of the scaled 3D digital model and the 2D digital image until the difference falls below a certain threshold, and
- implementing an optimization algorithm such as gradient descent to find the one or more scaling factors that minimize the difference between the geometric morphometrics of the 2D digital image and the 3D digital model.

6. The first computer-implemented method **(100)** of any one of claims 1 to 5, further comprising,
- using **(180)** the one or more scaling factors to adjust the size, shape, orientation and/or alignment of the 3D digital model to correspond with the size, shape, orientation and/or alignment of the 2D digital image, thereby generating an adjusted 3D digital model,
- positioning **(181)** the adjusted 3D digital model to align its landmarks with the corresponding landmarks of the 2D digital image, thereby generating a positioned adjusted 3D digital model, and
- superimposing **(182)** the positioned adjusted 3D digital model over the 2D digital image .

7. The first computer-implemented method **(100)** of claim 6, further comprising,
- deriving **(183)** a dental arch line on the positioned adjusted 3D digital model based on the first landmarks **(10, 11, 12)** and/or the first landmark-based geometric morphometrics,
- deriving **(184)** a dental arch line on the 2D digital image based on the second landmarks **(20, 21, 22)** and/or the second landmark-based geometric morphometrics, and
- adjusting **(185)** the derived dental arch line on the 2D digital image to match the derived dental arch line from the positioned adjusted 3D digital model.

8. A second computer-implemented method specifically intended **(200)** for digitally preparing natural teeth for prosthetic dental restorations, the second computer-implemented method **(200)** comprising the steps of,
- acquiring **(210)** a first positioned adjusted 3D digital model, as generated by the first computer-implemented method **(100)** of any one of claims 6 to 7, to represent one or more unprepared natural teeth of a patient along with the associated gum,
- acquiring **(220)** a second positioned adjusted 3D digital model, as generated by the first computer-implemented method **(100)** of any one of claims 6 to 7, that digitally represents proposed changes and/or improvements to the natural teeth, thereby illustrating an anticipated final outcome of the prosthetic dental restoration, and
- using **(230)** a Computer-Aided Design system, CAD, to digitally prepare the natural teeth for prosthetic dental restorations based on the first and second positioned adjusted 3D digital models, thereby creating a 3D digital dental model.

9. A third computer-implemented method specifically intended **(300)** for generating toolpaths to prepare natural teeth for prosthetic dental restorations, the third computer-implemented method **(300)** comprising the steps of,
- acquiring **(310)** a first positioned adjusted 3D digital model, as generated by the first computer-implemented method **(100)** of any one of claims 6 to 7, to represent one or more unprepared natural teeth of a patient along with the associated gum,
- acquiring **(320)** a second positioned adjusted 3D digital model, as generated by the second computer-implemented method **(200)** of claim 8, that digitally represents the prepared natural teeth of the patient along with the associated gum, wherein the prepared natural teeth correspond to the unprepared natural teeth whose surface has been prepared to coincide with one or more offset intrusion zones to increase its perceptibility, each offset intrusion zone representing an area where an inward offset penetrates the first positioned adjusted 3D digital model,
- generating **(330)** toolpaths based on the first and second positioned adjusted 3D digital models to prepare the natural teeth for prosthetic dental restorations, and
- providing **(340)** instructions or parameters to a Computer-Aided Manufacturing, CAM, system for preparing the natural teeth based on the generated toolpaths.

10. A fourth computer-implemented method specifically intended **(400)** for designing one or more dental prosthetics, the fourth computer-implemented method **(400)** comprising the steps of,
- using **(410)**, by a design system or device, a positioned adjusted 3D digital model, as generated by the second computer-implemented method **(200)** of claim 8, to design the dental prosthetics such that they fit within the patient's dentition, and
- providing **(420)** instructions or parameters to a manufacturing system or device for producing the one or more prosthetics based on the design.

11. A fifth computer-implemented method specifically intended **(500)** for generating customized dental implant surgery plans, the fifth computer-implemented method **(500)** comprising the steps of,
- using **(510)**, by a design system or device, a positioned adjusted 3D digital model, as generated by the first computer-implemented method **(100)** of any one of claims 6 to 7, to plan the surgeries, and
- determining **(520)** the appropriate size and position of dental implants based on this plan to ensure optimal placement within the patient's jaw, and
- providing **(530)** implant placement instructions or guidelines for a dental professional based on the plan.

12. A sixth computer-implemented method specifically intended **(600)** for creating orthodontic treatment plans, the sixth computer-implemented method **(600)** comprising the steps of,
- using **(610)**, by a design system or device, a positioned adjusted 3D digital model, as generated by the first computer-implemented method **(100)** of any one of claims 6 to 7, to create the treatment plans, and
- designing and sizing **(620)** orthodontic appliances based on the treatment plans to fit within the patient's dentition, and
- providing **(630)** specifications or parameters to a manufacturing system or device for producing the orthodontic appliances based on the design.

13. A seventh computer-implemented method specifically intended **(700)** for generating forensic dental analysis data, the seventh computer-implemented method **(700)** comprising the steps of,
- using **(710)**, by a design system or device, the positioned adjusted 3D digital model, as generated by the first computer-implemented method **(100)** of any one of claims 6 to 7, for the applications,
- generating **(720)** data for identifying individuals and analyzing bite marks based on the positioned adjusted 3D digital model, and
- providing **(730)** analysis data or identification markers for forensic professionals to support their investigations.

14. A computer-readable medium having stored thereon computer instructions which when executed, by a processor, perform one or more of the first, second, third, fourth, fifth, sixth and seventh computer-implemented methods **(100**, **200**, **300**, **400**, **500**, **600**, **700)** according to any one of claims 1 to 13.

15. A 3D Computer-Aided Design, CAD, system **(800)** comprising,
- one or more memories **(810)** for storing,
- one or more 2D digital images of dentitions of one or more patients,
- one or more 3D digital models of dentitions and jaws of the one or more patients, and
- executable instructions for performing one or more of the first, second, third, fourth, fifth, sixth and seventh computer-implemented methods **(100**, **200**, **300**, **400**, **500**, **600**, **700)** of any one of claims 1 to 13,
- one or more user interfaces **(820)**,
- one or more processors **(830)** coupled with the one or more memories **(810)** and the one or more user interfaces **(820),** and configured for executing the executable instructions.

16. An apparatus **(900)** specifically designed and built for estimating one or more scaling factors to map between a 2D digital image of a dentition and a 3D digital model of a dentition and jaw, the apparatus **(900)** comprising,
- a processor-based computing system **(910)** configured to acquire a 3D digital model of the dentition and jaw of the patient, and a virtual or synthetic 2D digital image of the dentition of a patient that accurately represents the size and dimensions of the patient's dentition ,
- a processor-driven CAD coordinate system **(920)** wherein the 2D digital image is aligned with the X and Y axes, and the 3D digital model is centered and aligned with the X, Y, and Z axes, with the frontal view of the dentition facing the positive Y axis,
- an image processor **(930)** configured to determine three or more first landmarks **(10, 11, 12)** on the dentition in the 3D digital model and three or more second landmarks **(20, 21, 22)** on the dentition in the 2D digital image , wherein the second landmarks **(20, 21**, **22)** are selected to correspond to the same anatomical locations as the first landmarks **(10, 11, 12)**,
- a processor-operated geometric morphometric calculator **(940)** designed to calculate first and second landmark-based geometric morphometrics based on the first landmarks **(10, 11, 12)** and second landmarks **(20, 21, 22)** respectively, and
- a processor-run scaling estimator **(950)** configured to estimate one or more scaling factors based on the comparison of the first and second landmark-based geometric morphometrics.

17. The apparatus **(900)** of claim 16, wherein the first landmarks **(10, 11, 12)** and second landmarks **(20, 21, 22)** comprise the cusp tips of the canines, the mesial and distal limits of the incisive edges, inter-canine width, and combinations thereof.

18. The apparatus **(900)** of any one of claims 16 to 17, wherein the processor-operated geometric morphometric calculator **(940)** is further configured to perform operations including calculating distances between landmarks, computing angles between landmarks, determining the area of polygonal regions defined by multiple landmarks, assessing the curvature of the dental arch at landmark locations, analyzing the relative alignment of landmarks, and combinations thereof.

19. The apparatus **(900)** of any one of claims 16 to 18, wherein the processor-run scaling estimator **(950)** is further configured to compare the first and second landmark-based geometric morphometrics by calculating one or more scaling factors that minimizes the differences between the,
- calculated distances between landmarks,
- computed angles between landmarks,
- determined areas of polygonal regions defined by multiple landmarks,
- assessed curvatures of the dental arch at landmark locations,
- analyzed relative alignments of landmarks, and
any combination thereof.

20. The apparatus **(900)** of claim of any one of claims 16 to 19, wherein the processor-run scaling estimator **(950)** is further configured to calculate the one or more scaling factors using one or more of the following methods:
- calculating an average ratio of corresponding landmark distances in the 2D digital image and the 3D digital model,
- applying a least squares method to minimize the sum of the squares of the differences between the observed and estimated values of the geometric morphometrics,
- training a machine learning model on a dataset of 2D and 3D images with known scaling factors to predict the one or more scaling factors for a new pair of 2D and 3D images,
- using iterative refinement to start with an initial estimate for the one or more scaling factors, then refine this estimate by comparing the geometric morphometrics of the scaled 3D digital model and the 2D digital image until the difference falls below a certain threshold, and
- implementing an optimization algorithm such as gradient descent to find the one or more scaling factors that minimize the difference between the geometric morphometrics of the 2D digital image and the 3D digital model.

21. The apparatus **(900)** of any one of claims 16 to 20, further comprising,
- a processor-model adjuster **(960)** configured to use the one or more scaling factors to adjust the size, shape, orientation and/or alignment of the 3D digital model to correspond with the size, shape, orientation and/or alignment of the 2D digital image, thereby generating an adjusted 3D digital model,
- a processor-controlled positioner **(970)** configured to align the landmarks of the adjusted 3D digital model with the corresponding landmarks of the 2D digital image, thereby generating a positioned adjusted 3D digital model, and
- a processor-guided superimposer **(980)** configured to overlay the positioned adjusted 3D digital model over the 2D digital image.

22. The apparatus **(900)** of claim 21, further comprising,
- a processor-operated dental arch line deriver **(990)** configured to derive,
- a dental arch line on the 2D digital image based on the second landmarks **20, 21, 22** and/or the second landmark-based geometric morphometrics, and
- a dental arch line on the positioned adjusted 3D digital model based on the first landmarks **10, 11, 12** and/or the first landmark-based geometric morphometrics, and
- a processor-run arch line adjuster **(991)** configured to adjust the derived dental arch line on the 2D digital image to match the derived dental arch line from the positioned adjusted 3D digital model.
